(19) Europäisches Patentamt
European Patent Office
Office européen des brevets

(11) Veröffentlichungsnummer: **0 176 884**
**B1**

# (12) EUROPÄISCHE PATENTSCHRIFT

(45) Veröffentlichungstag der Patentschrift: **07.12.88**

(51) Int. Cl.⁴: **B 01 F 17/54,** C 08 L 83/12

(21) Anmeldenummer: **85111899.2**

(22) Anmeldetag: **20.09.85**

(54) **Verwendung von Polyoxyalkylen-Polysiloxan-Copolymerisaten mit an Siliciumatomen gebundenen langkettigen Alkylresten als Emulgatoren zur Herstellung von W/O-Emulsionen.**

(30) Priorität: **03.10.84 DE 3436177**

(43) Veröffentlichungstag der Anmeldung: **09.04.86 Patentblatt 86/15**

(45) Bekanntmachung des Hinweises auf die Patenterteilung: **07.12.88 Patentblatt 88/49**

(84) Benannte Vertragsstaaten: **BE DE FR GB IT**

(56) Entgegenhaltungen:
**EP-A- 0 125 779**
**DE-B- 1 962 039**
**GB-A- 2 113 236**
**US-A- 4 047 958**

(73) Patentinhaber: **Th. Goldschmidt AG, Goldschmidtstrasse 100 Postfach 101461, D-4300 Essen 1 (DE)**

(72) Erfinder: **Hüttinger, Rudolf, Dr., Küppersheide 8, D-4300 Essen (DE)**
Erfinder: **Kollmeier, Hans-Joachim, Dr., Barkhorstrücken 27, D-4300 Essen (DE)**
Erfinder: **Langenhagen, Rolf-Dieter, Kampstrasse 5, D-4321 Hattingen-Niederwenigern (DE)**
Erfinder: **Walter, Alfred, Effmannstrasse 10, D-4300 Essen (DE)**
Erfinder: **Wolfes, Wilhelm, Dr., Ahornstrasse 45, D-4300 Essen (DE)**

EP 0 176 884 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung von an sich bekannten Copolymerisaten auf Polysiloxanbasis als Emulgatoren zur Hertellung von W/O-Emulsionen, deren ölige Phase aus Siliconöl besteht oder dieses enthält.

Zur Herstellung von Emulsionen des Typs W/O verwendet man Emulgatoren, die bevorzugt öllöslich oder in Öl dispergierbar sind und deren HLB-Wert meist < 8 ist. Dabei werden die Emulgatoren in der Regel in einer Menge von 10 bis 20 Gew.-%, bezogen auf Gewicht der öligen Phase, eingesetzt. Beispiele von solchen W/O-Emulgatoren sind insbesondere die Fettsäureester des Glycerins, des Polyglycerins oder des Sorbits sowie die Wollwachsalkohole. Als Fettsäurekomponenten werden meist Ölsäure oder Isostearinsäure verwendet.

Diese W/O-Emulsionen werden bevorzugt in der Kosmetik und Pharmazie, aber auch für technische Zwecke eingesetzt.

Besondere Schwierigkeiten treten nach dem Stand der Technik bei der Herstellung von W/O-Emulsionen auf, deren ölige Phase ganz oder teilweise aus Siliconöl besteht. Siliconöle lassen sich mit herkömmlichen Emulgatoren auf Polyolfettsäureesterbasis nur unbefriedigend emulgieren. Es wurden für besondere Probleme jeweils spezielle Rezepturen erarbeitet.

So sind aus der US-PS 4 268 499, Seite 1, Zeile 6, Antiperspirant-Zubereitungen in der Form von W/O-Emulsionen bekannt, die aus

a) 30 bis 60 Gew.-Teilen einer wässrigen Lösung eines adstringierenden Mittels, z.B. $AlCl_3$ als diskontinuierliche Phase,
b) 27 bis 67,5 Gew.-Teilen einer flüchtigen Flüssigkeit mit einem Siedepunkt unter 250 °C, z.B. einem cyclischen Dimethylsiloxan,
c) 0,5 bis 3 Gew.-Teilen eines W/O-Emulgators mit einem HLB-Wert von 2 bis 10,
d) 1 bis 5 Gew.-Teilen eines Polyoxyalkylen-Polysiloxan-Copolymerisats,
e) 1 bis 5 Gew.-Teilen eines W/O-Emulgators mit einem HLB-Wert von 11 bis 17,

wobei die Bestandteile a) bis e) 100 ergeben, bestehen.

Im Handel sind ferner zur Herstellung von W/O-Emulsionen, deren ölige Phase aus Siliconöl besteht oder dieses in überwiegender Menge enthält, Emulgatoren erhältlich, die aus einer Losung von Polyoxyalkylen-Polysiloxan-Copolymerisaten in einem cyclischen Siloxan bestehen. Diese Produkte sind z.B. unter der Bezeichnung DOW CORNING® Q2-3225C erhältlich. Ihre Anwendung ist in der Broschüre «A formulary of product application in skin care by DOW CORNING» beschrieben.

Diese Produkte können jedoch noch nicht in jeder Hinsicht befriedigen. Die W/O-Zubereitungen müssen lagerstabil sein, um eine ausreichende Lagerfähigkeit bei gleichbleibender Produktgüte zu gewährleisten. Die Emulsionen dürfen über einen Zeitraum von mehreren Monaten weder ihre Konsistenz verändern noch Öl- oder Wasseranteile ausscheiden. Dabei muss die Lagerstabilität über einen Bereich von −5° bis +40° beibehalten werden. Es bereitet jedoch insbesondere noch Schwierigkeiten, stabile W/O-Emulsionen mit Siliconölen als öliger kontinuierlicher Phase herzustellen, deren Siliconölgehalt über 20 Gew.-%, vorzugsweise über 30 Gew.-%, bezogen auf Gesamtgewicht der Emulsion, beträgt und/oder welche neben dem Siliconöl noch andere ölige Substanzen, z.B. Paraffinöle, Esteröle oder flüssige bzw. feste Wachse, wie pflanzliche, tierische oder mineralische Wachse, enthalten. Die unter Verwendung der Emulgatoren des Standes der Technik aus solchen Gemischen von Siliconöl und kohlenstofforganischen Ölen hergestellten W/O-Emulsionen zeigen in der Regel eine ungenügende Emulsionsstabilität und brechen innerhalb von Stunden oder Tagen. Häufig ist auch die Herstellung einer nur kurzzeitig stabilen Emulsion unmöglich.

Es sind bereits Copolymerisate bekannt, bei welchen an ein lineares Polysiloxan sowohl Polyoxyalkylengruppen als auch langkettige Kohlenwasserstoffgruppen gebunden sind. Die Herstellung solcher Verbindungen ist in den US-Patentschriften 3 234 252, 4 047 958, 3 427 271 und 2 846 458 beschrieben. Die Herstellung geschieht dabei vorzugsweise dadurch, dass man an ein Polydiorganosiloxan, welches SiH-Gruppen aufweist, ein Olefin mit z.B. 6 bis 18 Kohlenstoffatomen und einen Polyoxyalkylenether eines olefinisch ungesättigten Alkohols, z.B. den Polyoxyalkylenether des Allylalkohols, addiert, wobei man die Addition in Gegenwart eines Platin enthaltenden Katalysators vornimmt.

Diese Copolymerisate können nach der US-PS 4 381 241 als Emulgatoren zur Herstellung von W/O-Emulsionen von Salzlösungen in flüssigen Kohlenwasserstoffen verwendet werden, die als Bohrflüssigkeiten einsetzbar sind. Es findet sich jedoch kein Hinweis, dass derartige Verbindungen zur Herstellung von W/O-Emulsionen geeignet sein könnten, deren ölige Phase aus Siliconöl und gegebenenfalls weiteren kohlenstofforganischen Ölen oder Wachsen besteht.

Der Erfindung liegt die Aufgabe zugrunde, W/O-Emulsionen, deren ölige Phase aus Siliconöl besteht oder dieses enthält, mit möglichst geringen Emulgatormengen herzustellen. Die Emulsionen sollen hohe Stabilität aufweisen und diese auch bei thermischer Belastung beibehalten. Die Emulgatoren müssen dabei physiologisch unbedenklich sein, da der bevorzugte Anwendungsbereich der W/O-Emulsion in der Kosmetik und Pharmazie liegt.

Erfindungsgemäss können diese Aufgaben gelöst und zusätzliche Vorteile erzielt werden durch die Verwendung von Copolymerisaten der durchschnittlichen Formel

$$CH_3-\underset{CH_3}{\overset{CH_3}{Si}}O-\left[\underset{CH_3}{\overset{CH_3}{Si}}O-\right]_n\left[\underset{\underset{O-(C_2H_4O-)_x(C_3H_6O-)_yR}{(CH_2)_3}}{\overset{CH_3}{Si}}O-\right]_m\left[\underset{\underset{CH_3}{(CH_2)_p}}{\overset{CH_3}{Si}}O-\right]_o\underset{CH_3}{\overset{CH_3}{Si}}-CH_3 \qquad I$$

wobei R ein Alkylrest mit 1 bis 4 Kohlenstoffatomen oder ein Wasserstoffrest ist,

$n =$ 10 bis 200,
$m =$ 1 bis 25 und
$o =$ 1 bis 100 mit der Massgabe ist, dass im durchschnittlichen Molekül $o \geqq m$ und $3\,o < n$ ist,
$p =$ 7 bis 17 ist, und

das Molgewicht des Restes $(C_2H_4O-)_x(C_3H_6O-)_yR$ 250 bis 2000 beträgt, wobei x und y so gewählt sind, dass das Gewichtsverhältnis von Oxyethylen- zu Oxypropylengruppen 100:0 bis 20:80 beträgt,

als Emulgatoren zur Herstellung von W/O-Emulsionen, deren ölige Phase aus Siliconöl besteht oder dieses enthält, in Mengen von 0,3 bis 5 Gew.-%, bezogen auf Gesamtgewicht der Emulsion.

Vorzugsweise ist R ein Methyl- oder Wasserstoffrest.

Besonders bevorzugt sind Copolymerisate der Formel I, bei denen R ein Wasserstoffrest ist, $n =$ 25 bis 150, $o =$ 5 bis 50, $m =$ 1 bis 15 ist, wobei $o \geqq 2\,m$ und $3\,o < n$ ist. Bevorzugt ist das Gewichtsverhältnis von Oxyethylen- zu Oxypropylengruppen 40:60 bis 70:30. Das Molgewicht des Restes $(C_2H_4O-)_x(C_3H_6O-)_yR$ ist vorzugsweise 400 bis 1200.

Die Oxyethylen- und die Oxypropyleneinheiten können statistisch verteilt oder blockweise angeordnet sein.

Die Copolymerisate werden vorzugsweise in Mengen von 0,5 bis 2 Gew.-%, bezogen auf Gesamtgewicht der Emulsion, verwendet.

Ein wesentliches Merkmal der erfindungsgemäss zu verwendenden Copolymerisate besteht darin, dass im durchschnittlichen Molekül über eine SiC-Bindung sowohl ein oder mehrere langkettige Alkylreste als auch ein oder mehrere Polyoxylkylenreste bestimmter Zusammensetzung nebeneinander vorliegen. Dabei darf die Anzahl der Polyoxyalkylenreste allenfalls gleich der Anzahl der Einheiten mit langkettigen Alkylresten sein. Vorzugsweise ist die Anzahl der Einheiten mit langkettigen Alkylresten mindestens doppelt so gross wie die Anzahl der Einheiten mit Oxyalkylenresten. Hierdurch wird sichergestellt, dass das Verhältnis von hydrophilen und hydrophoben Anteilen im Molekül so ausgewogen ist, dass ein Optimum der W/O-Emulsionsstabilität erzielt wird.

Die Bedingung, dass die Anzahl der Dimethylsiloxyeinheiten grösser sein muss als die dreifache Anzahl der Siloxyeinheiten, welche langkettige Alkylreste tragen, ist von wesentlicher Bedeutung für die Verträglichkeit der Verbindungen der Formel I mit dem Siliconöl, welches Bestandteil der kontinuierlichen Phase der Emulsion ist. Die Emulgatoren der Formel I unterscheiden sich hierdurch wesentlich von den Emulgatoren der europäischen Patentanmeldung 0 125 779, welche der Bedingung entsprechen müssen, dass die Anzahl der Dimethylsiloxyeinheiten gleich oder kleiner als die dreifache Anzahl der Siloxyeinheiten mit langkettigen Alkylresten sein muss. Emulgatoren, welche dieser Bedingung entsprechen, zeigen nur eine geringe Verträglichkeit mit Siliconölen und sind wesentlich schlechter zur Herstellung von Wasser/Öl-Emulsionen, deren ölige Phase Siliconöl enthält, geeignet.

Die erfindungsgemäss zu verwendenden Emulgatoren können auch in Kombination mit den bekannten W/O-Emulgatoren, insbesondere Polyolfettsäureester, wie z.B. Ölsäure- oder Isostearinsäureester des Glycerins, Polyglycerins oder Sorbits sowie den Wollwachsalkoholen, verwendet werden. Es ist dem Fachmann bekannt, dass Emulgatorengemische häufig besonders stabile Emulsionen herzustellen gestatten. Das zweckmässig einzuhaltende Gewichtsverhältnis von erfindungsgemäss zu verwendenden Copolymerisaten und Fettsäureestern beträgt etwa 1:0,5 bis 1:2.

Die kontinuierliche ölige Phase kann ausschliesslich aus Siliconölen bestehen. Siliconöle im Sinne vorliegender Erfindung sind dabei flüssige bis hochviskose, insbesondere geradkettige oder cyclische, siliciumorganische Verbindungen, deren Si-Einheiten überwiegend difunktionell sind und die der Formel

$$\left[\underset{R^2}{\overset{R^1}{Si}}-O-\right]_p$$

entsprechen. Die Reste $R^1$ und $R^2$ können gleich oder verschieden sein und sind Kohlenwasserstoffreste, insbesondere Alkyl- oder Arylreste, besonders bevorzugt Methyl- oder Phenylreste. Ein Teil der Reste $R^1$, $R^2$ können Wasserstoffreste sein. p ist eine Zahl $\geqq 2$. Die Siliconöle können beliebige Endgruppen, insbesondere Gruppen der Formel $(R^1, R^2)_3SiO-$ oder Hydroxylgruppen, aufweisen. In den Siliconölen kön-

nen ın geringer Menge auch trifunktionelle Siliciumeinheiten der Formel $(R^1, R^2)SiO_{1,5}$– enthalten sein. Die Viskosität der Silicone liegt meist in einem Bereich von 0,5 mPa.s bis $3\cdot10^5$ mPa.s.

Die ölige Phase der W/O-Emulsionen kann neben dem Siliconöl kohlenstofforganische Öle oder Wachse enthalten. Als kohlenstofforganische Öle kommen insbesondere Mineralöle, tierische oder pflanzliche Öle in Frage. Beispiele hiervon sind Paraffinöle unterschiedlicher Viskositäten, Fischöle, Klauenöle, Knochenöle, Nerzöl, Olivenöl, Rizinusöl, Erdnussöl, Palmöl, Geeignet sind ferner flüssige Wachse, wie Jojobaöl oder synthetische Öle, wie Isopropylmyristat, Isopropylstearat, Laurinsäurehexylester, Ölsäureoleylester.

Den zu emulgierenden Ölen können auch Fette oder Wachse zugesetzt sein, wenn sie einen Schmelzpunkt oder Schmelzbereich < 100° aufweisen.

Zur Erhöhung der Kältestabilität können der Emulsion in an sich bekannter Weise Polyole, wie z.B. Glycerin, 1,2-Propylenglykol oder Sorbit, in Mengen von 0,5 bis 15 Gew.-%, bezogen auf Gesamtgewicht, zugesetzt werden.

Die Wärmestabilität kann in an sich bekannter Weise durch Zusatz von Elektrolyten, wie z.B. Natriumchlorid oder Magnesiumsulfat, oder durch Zusatz von Metallseifen, wie Calcium- oder Aluminiumstearat verbessert werden.

Der Gehalt der Emulsion an öliger kontinuierlicher Phase kann in weiten Grenzen schwanken. Er beträgt von 8 bis 50 Gew.-%, bezogen auf Gesamtgewicht der Zubereitung.

Die Herstellung der gewünschten W/O-Emulsion gelingt in an sich bekannter Weise. Sie ist nicht Gegenstand der Erfindung. Zweckmässig löst oder dispergiert man den Emulgator oder das Emulgatorengemisch in der öligen Phase. Enthält diese höherschmelzende Anteile, wie z.B. Bienenwachs, wird die ölige Phase über den Schmelzbereich dieser Bestandteile erwärmt. Das Wasser wird dann der öligen Phase unter gutem Rühren zugegeben. Weitere Bestandteile der Emulsion, wie Konservierungsmittel, Duft- oder Farbstoffe, Stabilisatoren, werden in der Regel der Phase zugegeben, in der sie löslich oder leicht dispergierbar sind.

Die mit den erfindungsgemäss zu verwendenden Copolymerisaten erhaltenen W/O-Emulsionen haben die geforderte hohe Stabilität und behalten diese über einen weiten Temperaturbereich bei relativ niedrigen Emulgatorkonzentrationen. Infolge des niedrigen Emulgatorgehaltes werden die Eigenschaften der öligen Phase nur entsprechend gering vom Emulgator beeinflusst. Dies ist insbesondere für kosmetische oder pharmazeutische Anwendungen von Bedeutung. Die mit den erfindungsgemäss zu verwendenden Emulgatoren hergestellten W/O-Emulsionen eignen sich auch für technische Zwecke, z.B. zur Verwendung als Pflegemittel und Polituren für Möbel, lackierte Bleche oder Fussböden.

In den folgenden Beispielen werden die Zusammensetzung von Emulsionen mit den erfindungsgemäss zu verwendenden Copolymerisaten, die Herstellung der Emulsionen und deren Eigenschaften gezeigt.

Zusammensetzung der Emulsionen

| | Emulsion<br>Bestandteile | 1<br>Gew.-% | 2<br>Gew.-% | 3<br>Gew.-% | 4<br>Gew.-% | 5<br>Gew.-% | 6<br>Gew.-% | 7<br>Gew.-% | 8<br>Gew.-% |
|---|---|---|---|---|---|---|---|---|---|
| | Emulgator I | 1,0 | – | – | – | – | – | – | – |
| | Emulgator II | – | 1,0 | – | – | – | – | – | 1,0 |
| | Emulgator III | – | – | 1,0 | – | – | – | – | – |
| | Emulgator IV | – | – | – | 1,0 | – | – | – | – |
| | Emulgator V | – | – | – | – | 1,0 | – | – | – |
| | Emulgator VI | – | – | – | – | – | 1,0 | 1,0 | – |
| | Triglycerintrioleat | 0,5 | 0,5 | 0,5 | 0,5 | 0,5 | 0,5 | – | 0,5 |
| | Octamethylcyclotetrasiloxan | 8,5 | 8,5 | 0,5 | 8,5 | 8,5 | 8,5 | 8,5 | 30,0 |
| A | Isopropylmyristat | 6,0 | 4,0 | 7,0 | 4,0 | 4,0 | 11,0 | 6,0 | – |
| | Paraffinöl DAB 8 | 5,0 | – | 6,0 | – | – | 6,0 | 5,0 | – |
| | flüssiges Esterwachs | 2,0 | – | 5,5 | – | – | – | 2,0 | – |
| | Vaseline DAB 8 | – | 5,0 | – | – | – | – | – | – |
| | Mikrowachs | – | – | 2,0 | – | – | 2,0 | – | – |
| | Polysiloxan-Polyoxyalkylen-Copolymerisat | – | – | – | 5,0 | 5,0 | – | – | 5,0 |
| | Maiskeimöl | – | – | – | – | 2,0 | – | – | – |
| | 3-(4-Methylbenzyliden)-campher | – | – | – | – | – | 3,0 | – | – |
| | Wasser | 70,0 | 79,0 | 75,5 | 79,0 | 72,0 | 66,0 | 70,5 | 56,5 |
| | NaCl | 2,0 | 2,0 | 2,0 | 2,0 | 2,0 | 2,0 | 2,0 | 2,0 |

Zusammensetzung der Emulsionen (Fortsetzung)

| | Emulsion Bestandteile | 1 Gew.-% | 2 Gew.-% | 3 Gew.-% | 4 Gew.-% | 5 Gew.-% | 6 Gew.-% | 7 Gew.-% | 8 Gew.-% |
|---|---|---|---|---|---|---|---|---|---|
| B | Glycerin | 5,0 | – | – | – | – | – | 5,0 | 5,0 |
| | 1,2-Propylenglykol | – | – | – | – | 5,0 | – | – | – |
| | | 100,0 | 100,0 | 100,0 | 100,0 | 100,0 | 100,0 | 100,0 | 100,0 |

Zusammensetzung der Emulgaroten I–VI

| | Indices in Formel I n | m | o | p | R | $(C_2H_4O-)_x(C_3H_6O-)_yR$ Molgewicht | Gewichtsverhältnis $C_2H_4O : C_3H_6O$ |
|---|---|---|---|---|---|---|---|
| Emulgator I | 38 | 3 | 7 | 17 | H | 490 | 20 : 80 |
| Emulgator II | 73 | 4 | 21 | 15 | H | 600 | 60 : 40 |
| Emulgator III | 92 | 5 | 26 | 15 | H | 550 | 60 : 40 |
| Emulgator IV | 148 | 8 | 42 | 11 | H | 400 | 60 : 40 |
| Emulgator V | 73 | 4 | 21 | 11 | H | 1200 | 80 : 20 |
| Emulgator VI | 80 | 3 | 18 | 17 | $CH_3$ | 700 | 100 : 0 |

Die Emulsionen 1 bis 5, 7 und 8 haben cremeartige Konsistenz; die Emulsion 6 ist flüssig. Die erhaltenen Emulsionen sind während einer Beobachtungszeit von 3 Monaten, bei 20 °C und 45 °C gelagert, stabil. Die Emulsionen zeigen nach Abkühlung auf −25 °C und darauffolgendem Erwärmen auf 20 °C keine Beeinträchtigung ihrer Stabilität. Die Emulsionen lassen sich auf der Haut gut verteilen und besitzen ein gutes Einziehvermögen.

Die Herstellung der Emulsionen erfolgt in einem Behälter aus Glas, der mit einem Rührer versehen ist. In den Behälter werden die Bestandteile A gegeben und gegebenenfalls aufgeschmolzen. Nach Anstellen des Rührers wird das Wasser, in welchem Kochsalz und gegebenenfalls Glycerin oder Propylenglykol gelöst sind, zu dem Gemisch aus Emulgator und Öl gegeben. Die erhaltene W/O-Emulsion wird gerührt bis sie Raumtemperatur annimmt.

**Patentanspruch**

Verwendung von Copolymerisaten der durchschnittlichen Formel

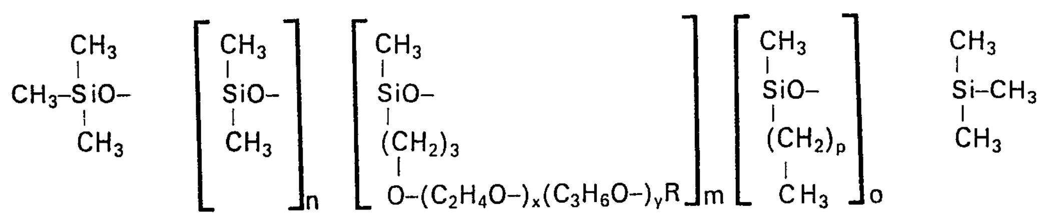

wobei

R ein Alkylrest mit 1 bis 4 Kohlenstoffatomen oder ein Wasserstoffrest ist,

n = 10 bis 200,

m = 1 bis 25 und

o = 1 bis 100 mit der Massgabe ist, dass im durchschnittlichen Molekül $o \geqq m$ und $3 o < n$ ist,

p = 7 bis 17 ist, und

das Molgewicht des Restes $(C_2H_4O-)_x(C_3H_6O-)_yR$ 250 bis 2000 beträgt, wobei x und y so gewählt sind, dass das Gewichtsverhältnis von Oxyethylen- zu Oxypropylengruppen 100:0 bis 20:80 beträgt,

als Emulgatoren zur Herstellung von W/O-Emulsionen, deren ölige Phase aus Siliconöl besteht oder dieses enthält, in Mengen von 0,3 bis 5 Gew.-%, bezogen auf Gesamtgewicht der Emulsion.

**Revendication**

Utilisation de copolymères ayant la formule moyenne suivante:

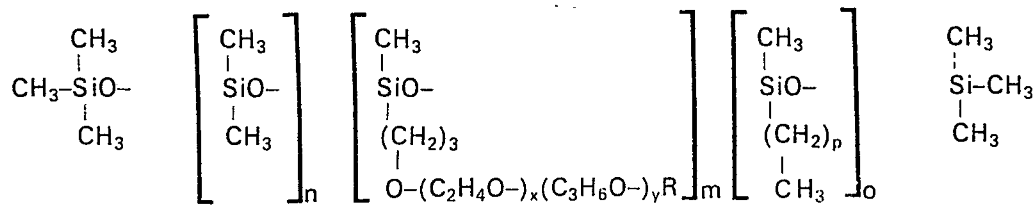

dans laquelle R est un radical alkyle ayant de 1 à 4 atomes de carbone, ou un radical hydrogène,

n = 10 à 200,
m = 1 à 25 und
o = 1 à 100 à la condition que, dans la molé cule moyenne, o≧ m et 3 o < n est,
p = 7 à 17 est, et

la masse moléculaire du radical $(C_2H_4O-)_x$-$(C_3H_6O-)_yR$ est de 250 à 2000, x et y étant choisis de façon que le rapport pondéral entre les groupes oxyéthylène .et oxypropylène soient compris entre 100:0 et 20:80, comme émulsifiants pour la préparation d'émulsions eau dans l'huile dont la phase huileuse est constituée d'une huile de silicone ou la contient, en des quantités de 0,3 à 5% en poids, par rapport au poids total de l'émulsion.

**Claim**

Use of copolymers of the average formula

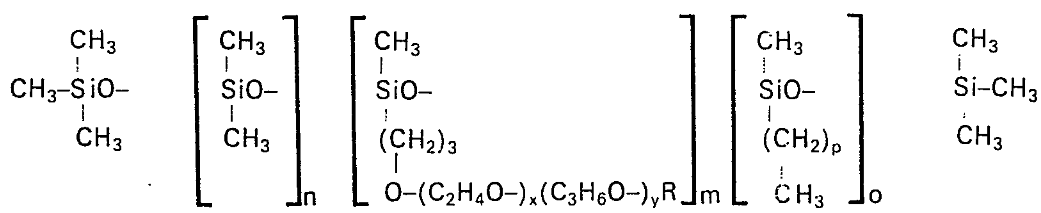

in which R is an alkyl radical having 1 to 4 carbon atoms or is a hydrogen radical,

n = 10 is 200,
m = 1 is 25 to
o = 1 is 100 with the proviso that, in the aver molecule, o≧ m and 3 o < n and,
p = 7 is 17 to, and

the molecular weight of the $(C_2H_4O-)_x$ $(C_3H_6O-)_yR$ radical is 250 to 2000, with x and y being selected so that the weight ratio between oxyethylene and oxypropylene groups is 100:0 to 20:80, as emulsifiers in the preparation of W/O emulsions whose oil phase comprises silicone oil or contains same, in amounts from 0,3 to 5% by weight, relative to the total weight of the emulsion.